# EUROPÄISCHE PATENTSCHRIFT

(11) **EP 2 748 142 B1**
(45) Veröffentlichungstag und Bekanntmachung des Hinweises auf die Patenterteilung: **05.08.2015**
(21) Anmeldenummer: 12746099.6
(22) Anmeldetag: 14.08.2012
(51) Int. Cl.: C07C 319/22, C07C 323/12

(54) **VERFAHREN ZUR HERSTELLUNG VON MERCAPTOALKYLCARBOXYLATEN**
PROCESS FOR PREPARING MERCAPTOALKYL CARBOXYLATES
PROCÉDÉ DE PRODUCTION DE MERCAPTOALKYLCARBOXYLATES

(30) Priorität: 24.08.2011 EP 11178664
(43) Veröffentlichungstag der Anmeldung: 02.07.2014
(73) Patentinhaber: BASF SE, 67056 Ludwigshafen (DE)
(72) Erfinder: GARLICHS, Florian, 67435 Neustadt (DE); BALDAMUS, Jens, 67067 Ludwigshafen (DE); KAIBEL, Gerd, 68623 Lampertheim (DE)
(86) Internationale Anmeldenummer: PCT/EP2012/065862
(87) Internationale Veröffentlichungsnummer: WO 2013/026739

(56) Entgegenhaltungen:
- WO-A1-2008/069904
- US-A- 4 615 836
- US-A- 5 916 987
- H. SELIGER, ET AL.: "Synthesis of aliphatic sulfenyl halides containing ester groups", SYNTHETIC COMMUNICATIONS, Bd. 10, Nr. 3, 1. Januar 1980 (1980-01-01) , Seiten 175-182, XP008149555, Marcel Dekker, New York, NY, US ISSN: 0039-7911, DOI: 10.1080/00397918008064219
- M. NAHMANY, ET AL.: "One-step tethering of omega-mercaptoalkyl function to alcohols", SYNTHESIS, Nr. 17, September 2006 (2006-09), Seiten 2841-2844, XP002670805, Georg Thieme Verlag, Stuttgart, DE ISSN: 0039-7881, DOI: 10.1055/s-2006-942516
- J.W.J. BOSCO, ET AL.: "Potassium fluoride assisted selective acetylation of alcohols with acetic acid", SYNTHETIC COMMUNICATIONS, Bd. 34, Nr. 15, 1. Januar 2004 (2004-01-01), Seiten 2849-2855, XP055022587, Marcel Dekker, New York, NY, US ISSN: 0039-7911, DOI: 10.1081/scc-200026245

## Beschreibung

Die Erfindung betrifft Verfahren zur Herstellung von Mercaptoalkylcaboxylaten durch eigenkatalytische Umsetzung von Carbonsäuren mit Mercaptoalkylalkoholen.

Die technisch bedeutendste Methode zur Herstellung von Carbonsäureestern ist die direkte Veresterung einer Carbonsäure mit einem Alkohol. Die dabei eingesetzten Carbonsäuren reagieren aufgrund der geringen Acidität nur sehr langsam. Stand der Technik ist daher der Zusatz von starken Säuren (wie beispielsweise Schwefelsäure, p-Toluolsulfonsäure, saure Ionenaustauscher und Titanalkoxylate) als Katalysator für die Veresterung. Die Veresterung stellt eine Gleichgewichtsreaktion dar. Um das Reaktionsgleichgewicht auf die Seite der Reaktionsprodukte zu verschieben, ist es daher üblich, entweder die Edukte im Überschuss vorzulegen oder die Produkte während der Reaktion aus dem Reaktionsgemisch zu entfernen. So wird beispielsweise das Reaktionswasser gasförmig aus dem Reaktionsgemisch entfernt, um bei der Veresterung das Gleichgewicht auf die Seite des Esters zu verschieben. Häufig wird dies unter Einsatz eines Schleppmittels erreicht.

Bei einer Azeotropdestillation werden die Edukte, Carbonsäure und Alkohol, üblicherweise zusammen mit Schleppmittel und Katalysator vorgelegt. Das Reaktionswasser wird dann solange zusammen mit dem Schleppmittel destillativ entfernt, meist unter Rückführung des Schleppmittels, bis ein ausreichender Reaktionsumsatz erreicht ist, und der Ester aus dem Reaktionsgemisch isoliert werden kann.

Bei Reaktivdestillationen wird üblicherweise die Carbonsäure mit dem Alkohol in einer mit Katalysator beschickten Reaktionszone zur Reaktion gebracht. Die entstehenden Produkte (Reaktionswasser und Carbonsäureester) werden dann als Kopf- bzw. als Sumpfprodukte der Kolonne abgetrennt.

Generell können Carbonsäureester auch durch Umesterung oder Umsetzung des Alkohols mit aktivierten Carbonsäurederivaten (beispielsweise Säurechloride oder Säureanhydride) hergestellt werden. Dies wird vor allem bei der Umsetzung von thermisch empfindlichen Substraten, bzw. bei der Herstellung von empfindlichen Produkten angewandt. Veresterungsverfahren ausgehend von aktivierten Carbonsäurederivate sind jedoch zumeist kostenintensiv und unwirtschaftlich.

Ester aus Carbonsäuren und Mercaptoalkylalkoholen, die Mercaptoalkylcarboxylate, insbesondere das Mercaptoethylpropionat, sind geeignet für die Verwendung als Molmassenregler bei Polymerisationsreaktionen, anstelle von sonst üblichen aber umweltbedenklichen langkettigen Mercaptanen (WO 2010/094641).

Bei der homogen säurekatalysierten Veresterung von Carbonsäuren mit Mercaptoalkylalkoholen (säurekatalysierte Azeotropveresterung) im Batch-Verfahren, beispielsweise mit p-Toluolsulfonsäure (US 5,916,987) als homogenen Katalysator kommt es jedoch zu einer verstärkten Bildung von unerwünschten Hochsiedern (Oligomere oder Polymere des Mercaptoalkylalkohols und/oder O,S-acylierte Verbindungen). Um die Bildung dieser Nebenprodukte zu vermeiden, muss die Reaktion bei hoher Verdünnung durchgeführt werden. Dies wiederum erhöht den Aufwand bei der Herstellung des Esters, da das zugesetzte Lösemittel nach der Reaktion aufwendig destillativ entfernt werden muss. Zusätzlich ist bei homogen katalysierter Veresterung eine aufwändige Abtrennung des Katalysators erforderlich. WO2008/069904 beschreibt die säurekatalysierte Herstellung eines Mercaptoalkylcarboxylaten ausgehend von einem Säureanhydrid als aktiviertes Carbonsäurederivat. Seliger und Görtz (Synthetic Communications (1980), 10:175-182) beschreiben die Azeotropveresterung bestimmter Carbonsäuren mit bestimmten Mercaptoalkylalkoholen auch ohne Katalysatorzusatz. Bosco et al. (Synthetic Communications (2004), 34; 2849-2855) beschreiben eine solche Azeotroveresterung in Gegenwart von Kalziumfluorid als Katalysator. Bei Nahmany und Melman (Synthesis, (2006), 17, 2841-2844) wiederum wird die Veresterung durch Zusatz von Dicyclohexylcarbodiimid erreicht.

Für die Herstellung von Mercaptoalkylcarboxylaten sind auch Verfahren beschrieben, bei denen die Veresterungsreaktion heterogen katalysiert wird (Firouzabadi et al., J Molecular Catalysis A: Chemical (2008), 289:61-68; Tayebee et al., Monatshefte Chemie (2006), 137:1063-1069; Pandey et al., J Molecular Catalysis A: Chemical (2006), 245:255-259; Kumar et al., Synlett (2001), 2:206-209, US 4,615,836). Bei der Verwendung eines sauren Heterogenkatalysators ist die Abtrennung des Katalysators von dem Produktgemisch deutlich einfacher und weniger aufwendig als bei der Verwendung von homogenen Säurekatalysatoren. Üblicherweise ist jedoch der Einsatz von aktivierten Carbonysäurederivaten, wie z.B. Carbonsäureanhydriden, notwendig oder es sind große Mengen des Katalysators, bzw. große Säureüberschüsse notwendig. Doch auch bei der heterogen säurekatalysierten Veresterung kommt es zu unerwünschten Nebenprodukten wie beispielsweise Oligomere oder Polymere des Mercaptoalkylalkohols und/oder O,S-acylierte Verbindungen. Üblicherweise müssen die so gewonnenen Ester daher destillativ gereinigt werden. Teilweise fallen die Nebenprodukte in Form von Feststoffen an, was verfahrenstechnisch besonders nachteilig ist.

Als der Erfindung zugrunde liegende Aufgabe kann daher erachtet werden, Mercaptoalkylcarboxylate durch Veresterung von Carbonsäuren mit Mercaptoalkylalkoholen herzustellen und dabei die Nachteile des Standes der Technik, insbesondere die Bildung von unerwünschten Nebenprodukten, wie sie bei den homogen oder heterogen säurekatalysierten Veresterungen zu beobachten ist, zu vermeiden. Diese Aufgabe wird gelöst durch die im Folgenden beschriebenen und durch die beanspruchten Ausführungsformen.

Entsprechend betrifft die vorliegende Erfindung ein Verfahren zur Herstellung von Mercaptoalkylcarboxylaten durch eigenkatalytische Veresterung von Carbonsäuren mit Mercaptoalkylalkoholen mittels Reaktivdestillation in Abwesenheit von zusätzlichem wirksamen Säurekatalysator (homogen oder heterogen).

Ein zusätzlicher wirksamer Säurekatalysator (homogen oder heterogen) im Sinne der Erfindung ist eine Säure (Lewis- oder Brønsted-Säure), die nicht identisch ist mit der zu veresternden Carbonsäure, und die unter den gegebenen Veresterungsbedingungen und in der zugesetzten Menge bzw. im zugesetztem Anteil gemittelt über den gesamten Veresterungsansatz, eine Erhöhung der Geschwindigkeit der Veresterung der Carbonsäure mit dem Mercaptoalkylalkohol um mindestens 50 %, um mindestens 100 %, oder um mindestens 200 % bewirkt, verglichen mit dem ansonsten identischen Veresterungsansatz ohne Zusatz einer Säure (zusätzlich zu der zu veresternden Carbonsäure). Die Veresterungsbedingungen sind insbesondere charakterisiert durch die Art und die Menge bzw. den Anteil der eingesetzten Edukte, Carbonsäure und Mercaptoalkylalkohol, die Temperatur und dem Druck, und die Art und die Menge bzw. den Anteil eines optional eingesetzten Lösemittels. Die Geschwindigkeit der Veresterung ist der Grenzwert der Geschwindigkeit der Bildung des Veresterungsprodukts, Mercaptoalkylcarboxylat, für den Beginn der Umsetzung. Er lässt sich ermitteln als Steigung der Tangente an der Messung der Esterkonzentration gegen die Zeit im Zeitpunkt des Beginns der Veresterungsreaktion. Die Geschwindigkeit der Veresterung ist somit die zeitliche Ableitung der Funktion von Esterkonzentration gegen die Zeit im Zeitpunkt des Beginns der Veresterung. Abwesenheit von zusätzlichem wirksamen Säurekatalysator (homogen oder heterogen) im Sinne der Erfindung liegt somit auch dann vor, wenn zwar dem Reaktionsgemisch eine zusätzliche Säure hinzugefügt ist, diese aber unter den Reaktionsbedingungen keine Erhöhung der Veresterungsgeschwindigkeit in dem oben genannten Ausmaß bewirkt, oder diese in einer zu geringen Menge bzw. einem zu geringen Anteil zugesetzt ist, um eine solche Erhöhung der Veresterungsgeschwindigkeit zu bewirken.

Gegenstand der Erfindung ist somit insbesondere ein Verfahren zur Herstellung von Mercaptoalkylcarboxylaten durch Veresterung aus den Edukten Carbonsäure und Mercaptoalkylalkohol, dadurch gekennzeichnet, dass die Veresterung in Abwesenheit von zusätzlichem wirksamen Säurekatalysator durchgeführt wird, wobei besagter zusätzlicher wirksamer Säurekatalysator eine Menge einer Säure ist, die eine Erhöhung der Geschwindigkeit der besagten Veresterung gemittelt über den gesamten Veresterungsansatz um mindestens 50 %, um mindestens 100 %, oder um mindestens 200 % bei ansonsten identischen Veresterungsbedingungen bewirkt, verglichen mit der Veresterung der Carbonsäure mit dem Mercaptoalkylalkohol ohne zusätzliche Säure, wobei die Veresterung als Reaktivdestillation durchgeführt, bei der
a) die Edukte Carbonsäure und Mercaptoalkylalkohol als kontinuierlicher Strom der Reaktionszone einer Rektifikationskolonne zugeführt werden,
b) die Edukte in der Rektifikationskolonne zu den Reaktionsprodukten Mercaptoalkylcarboxylat und Reaktionswasser reagieren,
c) das Reaktionsgemisch in der Rektifikationskolonne destillativ aufgetrennt wird, und
d) eine Mercaptoalkylcarboxylat enthaltende Fraktion über Sumpf und eine Reaktionswasser enthaltende Fraktion über Kopf aus der Rektifikationskolonne abgeführt werden.

Die Menge bzw. der Anteil an gebildetem Mercaptoalkylcarboxylat im Verlauf der Veresterungsreaktion kann bestimmt werden mittels GC- oder HPLC-Analyse, insbesondere unter Einsatz von internen oder externen Standards auf die Edukte und Produkte der Veresterungsreaktion.

Grundsätzlich kann eine Veresterung diskontinuierlich (batch-Verfahren) als Azeotropdestillation durchgeführt werden. Das erfindungsgemäße Verfahren wird als Reaktivdestillation durchgeführt. In diesem Fall werden die Edukte, Mercaptoalkylalkohol und Carbonsäure, üblicherweise in einem im Wesentlichen stöchiometrischen Verhältnis von beispielsweise 0,8 bis 1,2 für Mercaptoalkylalkohol-Molmenge zu Carbonsäure-Molmenge, kontinuierlich einer Kolonne (Rektifikationskolonne mit Reaktionszone) zugeführt. Das entstehende Reaktionswasser wird über Kopf und das entstandene Mercaptoalkylcarboxylat über Sumpf abgezogen. Die Durchführung des Verfahrens als Reaktivdestillation, mit kontinuierlicher Prozessführung bei kontinuierlicher Abtrennung des Reaktionswassers und des Reaktionsproduktes, ermöglicht günstige Gesamtumsätze für die Reaktion.

Das erfindungsgemäße Verfahren zur Herstellung von Mercaptoalkylcarboxylaten wird somit als Reaktivdestillation durchgeführt, bei der
a) die Edukte Carbonsäure und Mercaptoalkylalkohol als kontinuierlicher Strom der Reaktionszone einer Rektifikationskolonne zugeführt werden,
b) die Edukte in der Rektifikationskolonne zu den Reaktionsprodukten Mercaptoalkylcarboxylat und Reaktionswasser reagieren,
c) das Reaktionsgemisch in der Rektifikationskolonne destillativ aufgetrennt wird, und
d) eine Mercaptoalkylcarboxylat enthaltende Fraktion über Sumpf und eine Reaktionswasser enthaltende Fraktion über Kopf aus der Rektifikationskolonne abgeführt werden.

Bevorzugt werden alle Schritte des Verfahrens kontinuierlich betrieben.

In einer Ausführungsform der Erfindung wird die Veresterung in einem Verweilzeitbehälter in Teilumsatz durchgeführt. Die Verweilzeit ist vorzugsweise so zu wählen, dass die bei der Gleichgewichtsreaktion anfänglich schnelle Reaktionsgeschwindigkeit abgedeckt wird. Vorzugsweise ist die Verweilzeit so einzustellen, dass unter den gegebenen Reaktionsbedingungen (insbesondere der eingestellten Temperatur, sowie Auswahl der Edukte) 5 bis 50 %, bevorzugt 10 bis 30 %, besonders bevorzugt 15 bis 25 % der Gleichgewichtskonzentration des Esters gebildet werden. Die Verweilzeit ergibt sich als Quotient aus dem Volumen des Gesamtreaktionsraums durch die Gesamtflussrate der dem Gesamtreaktionsraum zugeleiteten Ströme, also der neu eingebrachten Carbonsäure und des neu eingebrachten Mercaptoalkylalkohols und der ggf. zusätzlich zugeführten Löse- und/oder Schleppmittel. Der Gesamtreaktionsraum ist der Reaktionsraum der Vorrichtung in dem die Edukte zu dem Produkt verestern können, sie wird gebildet von dem Reaktionsraum des Verweilzeitbehälters und der Reaktionszone der Rektifikationskolonne, sowie auch der Verbindungsleitungen zwischen Verweilzeitbehälter und Rektifikationskolonne. Üblicherweise macht der Reaktionsraum des Verweilzeitbehälters den überwiegenden Teil des Gesamtreaktionsraums aus, vorzugsweise mindestens 60 %, besonders bevorzugt mindestens 80 %, insbesondere mindestens 90 %. Daher kann die Verweilzeit üblicherweise auch recht gut bestimmt werden als Quotient aus dem Volumen des Reaktionsraums des Verweilzeitbehälters durch die Gesamtflussrate der in den Gesamtreaktionsraum zugeleiteten Ströme. Die Verweilzeit lässt sich somit insbesondere durch die Dimensionierung des Verweilzeitbehälters im Verhältnis zu dem gewünschten Durchsatz einstellen. Der Verweilzeitbehälter verfügt vorzugsweise über eine Rühreinrichtung, einen Umpumpkreislauf und/oder eine Temperierung. Die Reaktion im Verweilzeitbehälter wird vorzugsweise bei einer Temperatur von 80 bis 200 °C, besonders bevorzugt bei 100 bis 160 °C, insbesondere bei 100 bis 140 °C durchgeführt. Der Absolutdruck im Verweilzeitbehälter liegt bevorzugt bei 0,5 bis 16 bar, besonders bevorzugt bei 0,9 bis 6 bar. Der Druck im Verweilzeitbehälter ist dabei mindestens gleich dem Dampfdruck des Reaktionsgemisches bei der gewählten Temperatur. Bevorzugt ist die Temperatur im Verweilzeitbehälter so gewählt, so dass sie der Temperatur an der Zulaufstelle der Kolonne entspricht bzw. leicht höher ist, aber maximal der Temperatur im Sumpf der Kolonne entspricht. Der Reaktionsraum des Verweilzeitbehälters steht im Volumenaustausch mit der Reaktionszone einer Rektifikationskolonne mit Abtriebs- und Auftriebsteil. In der Rektifikationskolonne werden die Produkte über Kopf bzw. Sumpf abgetrennt. Als Seitenabzug der Kolonne wird ein produktabgereicherter Strom erhalten. In einer bevorzugten Variante wird dieser Strom in den Verweilzeitbehälter zurückgeführt. Der Einsatz eines Verweilzeitbehälters bei der Durchführung der erfindungsgemäßen Reaktivdestillation ermöglicht vergleichsweise große Mengen an Edukt der Veresterungsreaktion auszusetzen, während die Abtrennung der Produkte (Ester und Reaktionswasser) in einer hinsichtlich des Mengendurchsatzes vergleichsweise klein dimensionierten und damit günstigen Rektifikationskolonne erfolgen kann. Der Einsatz des Verweilzeitbehälters bewirkt, dass die entsprechende Verweilzeit nicht in der Reaktionszone der Rektifikationskolonne realisiert werden muss, mit der Folge, dass die Rektifikationskolonne vergleichsweise klein dimensioniert werden kann.

Das erfindungsgemäße Verfahren zur Herstellung von Mercaptoalkylcarboxylat wird somit in einer bevorzugten Ausführungsform als Reaktivdestillation in Verbindung mit einem Verweilzeitbehälters durchgeführt, bei der
a) die Edukte, Carbonsäure und Mercaptoalkylalkohol, als kontinuierliche Ströme dem Gesamtreaktionsraum, umfassend den Reaktionsraum des Verweilzeitbehälters und die Reaktionszone der Rektifikationskolonne, zugeführt werden,
b) die Edukte in dem Gesamtreaktionsraum in Teilumsatz zu Mercaptoalkylcarboxylat und Reaktionswasser reagieren,
c) das Reaktionsgemisch aus Schritt b), umfassend Mercaptoalkylcarboxylat und Reaktionswasser als Produkte der Veresterung sowie unumgesetzte Edukte, vom Verweilzeitbehälter der Rektifikationskolonne zugeführt wird,
d) das Mercaptoalkylcarboxylat und das Reaktionswasser in der Rektifikationskolonne destillativ aus dem Reaktionsgemisch abgetrennt und über Sumpf bzw. über Kopf aus der Rektifikationskolonne abgeführt werden, während produktabgereichertes Reaktionsgemisch als Seitenabzug der Rektifikationskolonne abgeführt wird, und
e) das als Seitenabzug der Rektifikationskolonne abgeführte produktabgereichertes Reaktionsgemisch aus Schritt d) in den Verweilzeitbehälter zurückgeführt wird.

Dabei können ein oder mehrere produktabgereicherte Fraktionen als Seitenabzug bzw. Seitenabzüge aus der Rektifikationskolonne abgeführt werden, abhängig von den jeweiligen Siedepunkten der Edukte und Produkte unter den Destillationsbedingungen. Bevorzugt werden alle Schritte des Verfahrens kontinuierlich betrieben. Insbesondere erfolgt die Zuführung des Reaktionsgemisches aus dem Verweilzeitbehälter in die Rektifikationskolonne (Schritt c) und die Zurückführung der produktabgereicherte Fraktion bzw. produktabgereicherten Fraktionen als Seitenabzug bzw. Seitenabzüge aus der Rektifikationskolonne in den Verweilzeitbehälter (Schritt e) als kontinuierliche Ströme. Die destillative Fraktionierung in der Rektifikationskolonne erfolgt bevorzugt bei reduziertem Druck, beispielsweise bei einem Absolutdruck von 1 bis 800 mbar, typischerweise bei einem Absolutdruck von 10 bis 500 mbar. Unter solchen Bedingungen kann die Destillation bei geringerer Sumpftemperatur erfolgen, wodurch die Bildung von unerwünschten Nebenprodukten zusätzlich reduziert werden kann. Die destillative Fraktionierung kann aber auch bei Normaldruck oder erhöhtem Druck durchgeführt werden.

Je nach Wahl der Edukte kann das am Kopf der Kolonne abzuziehende Reaktionswasser azeotrop gebundenes Edukt mitführen. In einer bevorzugten Variante wird daher in solchen Fällen das Kopfprodukt der Kolonne mit einem geeignetem Hilfsstoff (z.B. Cyclohexan, Toluol) vermischt und einem Phasentrenngefäß zugeführt, sodass das Gemisch in eine flüssige, überwiegend Hilfsstoff und Edukt enthaltende Oberphase und eine überwiegend Wasser enthaltende Unterphase zerfällt. Die Wasserphase wird ausgeschleust, die Hilfsstoff/Edukt-Phase auf den Kopf der Kolonne zurückgeführt.

Für das erfindungsgemäße Veresterungsverfahren werden beispielsweise verzweigte oder unverzweigte Carbonsäuren mit 2 bis 8 C-Atomen, bevorzugt mit 2 bis 5 C-Atomen eingesetzt. Geeignete Carbonsäuren sind beispielsweise Essigsäure, Propionsäure, Buttersäure, Isobuttersäure, Valeriansäure, Isovaleriansäure, Capronsäure, Önanthsäure oder Caprylsäure.

Weiter werden für das erfindungsgemäße Veresterungsverfahren beispielsweise verzweigte oder unverzweigte Mercaptoalkylalkohole mit 2 bis 4 C-Atomen eingesetzt. Geeignete Mercaptoalkylalkohole sind beispielsweise 2-Mercaptoethanol, 2-Mercaptopropanol, 3-Mercaptopropanol, 1-Mercapto-2-propanol, 2-Mercaptobutanol, 3-Mercaptobutanol, 4-Mercaptobutanol, 1-Mercapto-2-butanol, 1-Mercapto-3-butanol, 2-Mercapto-3-butanol oder Mercaptoisobutanol.

Bei der Veresterung von Carbonsäuren mit 2 bis 8 C-Atomen, insbesondere von Carbonsäuren mit 2 bis 5 C-Atomen, mit Mercaptoalkylalkoholen mit 2 bis 4 C-Atomen in einer Reaktivdestillation können, aufgrund der unterschiedlichen Siedepunkte von Reaktionswasser, Edukten und Ester, die entstandenen Produkte (Ester und Reaktionswasser) in günstiger Weise von den nicht umgesetzten Edukten (Mercaptoalkylalkohol und Carbonsäure) abgetrennt werden.

Eine bevorzugte Ausführungsform des erfindungsgemäßen Verfahrens betrifft die Veresterung von Propionsäure mit 2-Mercaptoethanol zu Mercaptoethylpropionat.

Die erfindungsgemäße Veresterung von Carbonsäure mit Mercaptoalkylalkohol erfolgt vorzugsweise bei einer Temperatur von 80 bis 200 °C, bevorzugt bei 100 bis 160 °C, insbesondere bei 100 bis 140 °C und bei einem Absolutdruck von 0,5 bis 16 bar, bevorzugt bei 0,9 bis 6 bar.

Die erfindungsgemäße Veresterung von Carbonsäure mit Mercaptoalkylalkoholen kann in Gegenwart von Schleppmittel durchgeführt werden. Schleppmittel im Sinne der Erfindung umfassen keine Säuren. Bevorzugte Schleppmittel sind aromatische und /oder aliphatische Kohlenwasserstoffe, bspw. Toluol, Xylol, Cyclohexan, und/oder Cycloheptan, insbesondere Toluol. Dabei wird die erfindungsgemäße Veresterung vorzugsweise in Gegenwart von maximal 5 Gew.-% Schleppmittel bezogen auf die Gesamtmenge der Edukte (Carbonsäure und Mercaptoalkylalkohol) durchgeführt. Die erfindungsgemäße Veresterung kann auch in Gegenwart von Lösemitteln durchgeführt werden. Lösemittel im Sinne der Erfindung umfassen keine Säuren. Lösemittel können auch als Schleppmittel wirken und umgekehrt. Dabei wird die erfindungsgemäße Veresterung vorzugsweise in Gegenwart von maximal 5 Gew.-% Löse- und/oder Schleppmittel bezogen auf die Gesamtmenge der Edukte (Carbonsäure und Mercaptoalkylalkohol) durchgeführt. In einer Ausführungsform wird die erfindungsgemäße Veresterung als Reaktivdestillation ohne Zusatz von Löse- und /oder Schleppmittel durchgeführt.

Es wurde beobachtet, dass bei der erfindungsgemäßen Veresterung von Mercaptoalkylalkoholen mit Carbonsäuren ohne zusätzlichen wirksamen Säurekatalysator (homogen oder heterogen) die Bildung an unerwünschten Nebenprodukten deutlich verringert werden konnte. Bei den Nebenprodukten handelt es sich insbesondere um O,S-acylierte oder oligomere Verbindungen des Mercaptoalkylalkohols. Da diese Nebenprodukte in der Regel als unlösliche Feststoffe ausfallen, sind sie für die technische Durchführung des Verfahrens besonders problematisch. Die mit dem Verzicht auf den Zusatz wirksamer Säurekatalysatoren einhergehende Verringerung der Reaktionsgeschwindigkeit kann erfindungsgemäß kompensiert werden durch die Durchführung der Veresterung mittels Reaktivdestillation insbesondere unter Einsatz eines Verweilzeitbehälters für die Umsetzung. Selbst unter Erhöhung der Reaktionstemperatur und Verlängerung der Reaktionszeit (Verweilzeit), sodass ein mit einer katalytischen Umsetzung von Mercaptoalkylalkoholen mit Carbonsäuren vergleichbarer Umsatz erreicht wurde, und gleichzeitig die bei der katalytischen Umsetzung beobachtete Bildung von Nebenprodukten vermieden wurde. Das erfindungsgemäße Verfahren bietet einen effektiven Ersatz für die katalytische Veresterung von Mercaptoalkylalkoholen mit Carbonsäuren unter Vermeidung oder zumindest Verringerung der ansonsten auftretenden Nebenproduktbildung.

### Beschreibung der Abbildung

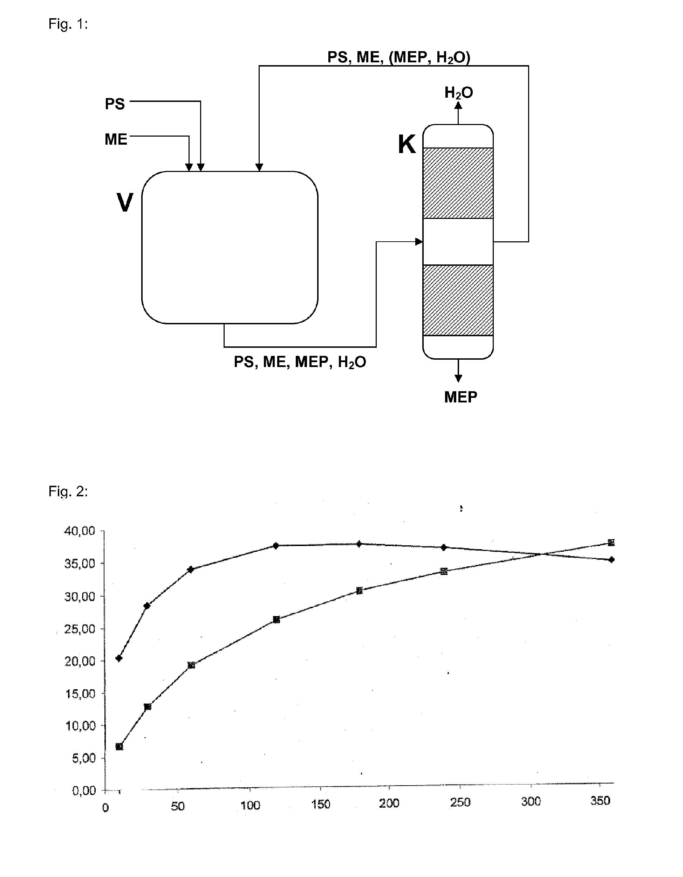

Fig. 1 veranschaulicht das erfindungsgemäße Verfahren am Beispiel einer Reaktivdestillation zur Veresterung von Propionsäure (PS) mit Mercaptoethanol (ME) zu dem Ester Mercaptoethylpropionat (MEP). Die Edukte Propionsäure (PS) und Mercaptoethanol (ME) werden in den Verweilzeitbehälter (V) geleitet. Der Verweilzeitbehälter kann auf eine Veresterungstemperatur temperiert und mit einer Rühreinrichtung ausgestattet sein. Dort reagieren der Alkohol und die Carbonsäure zu dem Ester unter Bildung von Reaktionswasser (H₂O). In Abhängigkeit von der Verweilzeit kann die Gleichgewichtskonzentration der Veresterungsprodukte mehr oder weniger vollständig erreicht werden. Die Verweilzeit ergibt sich aus dem Zustrom der Edukte (inkl. der zurückgeführten Edukte) und dem Volumen des Verweilzeitbehälters. Das Reaktionsgemisch (PS, ME, MEP, H₂O) aus dem Verweilzeitbehälter (V) wird dann weiter zur Reaktivdestillation in eine Kolonne (K) geleitet. In der Kolonne (K) werden die Komponenten des Reaktionsgemisches aufgetrennt. Während das Veresterungsprodukt, Mercaptoethylpropionat (MEP), über Sumpf und das Reaktionswasser (H₂O) über Kopf abgetrennt werden, wird ein produktabgereicherter Strom (PS, ME, (MEP, H₂O)) als Seitenabzug der Kolonne (K) entnommen und dem Reaktionsgemisch im Verweilzeitbehälter (V) zurückgeführt. Durch die kontinuierliche Prozessführung der Reaktivdestillation in Kombination mit der Nutzung eines Verweilzeitbehälters kann der Nachteil der vergleichsweise geringen Reaktionsgeschwindigkeit der Veresterungsreaktion bei Abwesenheit eines Säurekatalysators kompensiert werden.

Fig. 2 zeigt den Ausbeuteverlauf für die Veresterung von Propionsäure mit Mercaptoethanol. Gemessen wurde mittels gaschromatographischer Analyse (GC-Analyse) der Anteil von Mercaptoethylpropionat in Gew.-% (y-Achse) gegen die Reaktionszeit in min (x-Achse). Dargestellt sind der Ausbeuteverlauf für eine Veresterung ohne Zusatz von Katalysator (Bsp. 1; -■-) und der Ausbeuteverlauf für eine Veresterung unter Zusatz eines heterogenen Säurekatalysators (Vergleichsbsp. 1; -◆-).

### Ausführungsbeispiele

Die Erfindung wird nun durch die nachfolgenden, nichtlimitierenden Beispiele näher erläutert.

### Beispiel 1

Veresterung von Propionsäure mit Mercaptoethanol ohne Zusatz von Säurekatalysator und ohne Schleppmittel

In einem 250 ml Vierhalskolben wurden 78,1 g Mercaptoethanol (1 mol) und 74,1 g Propionsäure (1 mol) zusammengegeben und auf 140 °C erhitzt. Durch das gebildete Reaktionswasser sankt die Innentemperatur während der Reaktion auf 120 °C ab. Die Ausbeute an Mercaptoethylpropionat im Reaktionsverlauf wurde durch GC-Messungen während der Reaktion verfolgt und ist in Fig. 2 dargestellt. Nach 480 min wurde die Reaktionsmischung abgekühlt. Nach dem Abkühlen lag die Reaktionsmischung als klare, farblose Flüssigkeit vor. Die GC-Analyse des Rohprodukts ergab eine Zusammensetzung von 26,6 Gew.-% Propionsäure (entspr. 37,6 g), 30,2 Gew.-% Mercaptoethanol (entspr. 42,6 g), 37,1 Gew.-% Mercaptoethylpropionat (entspr. 52,3 g, 39,0 % der theoretischen Ausbeute) und 0,64 FI-% Hochsieder (FI-% steht für Flächenprozent des Peaks von dem GC-Chromatogramm).

### Vergleichsbeispiel 1

Veresterung von Propionsäure mit Mercaptoethanol ohne Schleppmittel aber unter Zusatz eines heterogenen Säurekatalysators

In einem 250 ml Vierhalskolben wurden 78,1 g Mercaptoethanol (1 mol) und 74,1 g Propionsäure (1 mol) zusammengegeben und 5 Gew.-% des Heterogenkatalysator Amberlyst® 15 (von Rohm and Haas Company) zugegeben. Es wurde auf 80 °C erhitzt. Die Ausbeute an Mercaptoethylpropionat im Reaktionsverlauf wurde durch GC-Messungen während der Reaktion verfolgt und ist in Fig. 2 dargestellt. Während der Reaktion kam es zu Bildung von oligomeren Nebenprodukten, die als Feststoff ausfielen. Die Bildung dieses Feststoffs begründet auch den Abfall der Esterausbeute nach einer Reaktionszeit von etwa 120 min, wie in Fig. 2 zu erkennen.

Durch den Zusatz des heterogenen Säurekatalysators wird die Reaktionsgeschwindigkeit der Veresterung zwar deutlich erhöht verglichen mit einem Ansatz ohne zusätzlichen Katalysator (Bsp. 1), doch es kommt auch zur verstärkten Bildung von unerwünschten Nebenprodukten (Bildung von Feststoff).

### Vergleichsbeispiel 2

Veresterung von Propionsäure mit Mercaptoethanol in Gegenwart eines sauren Katalysators und eines Schleppmittels (Toluol)

In einem 500 ml Planschliffreaktor wurden die Reaktionsgemische A, B und C entsprechend den Angaben in Tabelle 1 vorgelegt. Die Reaktionsmischung wurde jeweils auf Rückfluss erhitzt und das gebildete Reaktionswasser wird am Phasenscheider abgetrennt (90 min bei Rückfluss bei Variante A und B bzw. 150 min bei Rückfluss bei Variante C). Bei den Varianten A und B wurde p-Toluolsulfonsäure als homogener Säurekatalysator verwendet, während bei Variante C der heterogene Säurekatalysator Amberlyst 15 verwendet wurde. Bei allen drei Varianten wurden die Edukte mit einer großen Menge an Schleppmittel (Toluol) verdünnt (25 Gew.-% bezogen auf die Gesamtmenge der Edukte bei Variante A bzw. 150 Gew.-% bezogen auf die Gesamtmenge der Edukte bei Variante B und C).

**Tabelle 1:**

| | Reaktiongemisch A | Reaktiongemisch B | Reaktiongemisch C |
|---|---|---|---|
| Mercaptoethanol | 257,8 g (3,3 mol) | 117,2 g (1,5 mol) | 117,2 g (1,5 mol) |
| Propionsäure | 244,5 g (3,3 mol) | 111,1 g (1,5 mol) | 111,1 g (1,5 mol) |
| saurer Katalysator | p-Toluolsulfonsäure | | Amberlyst 15 |
| | 6,3 g (0,033 mol) | 2,9 g (0,015 mol) | 4,0 g |
| Toluol | 125,6 g | 342,5 g | 342,5 g |

Nach dem Abkühlen lag das Reaktionsgemisch von Variante A und B als klare, fast farblose Lösung vor, allerdings hatte sich bei Variante A Feststoff gebildet. Bei Variante C lag das Reaktionsgemisch nach dem Abkühlen als milchig trübe Lösung mit weißem, amorphem Feststoffanteil vor. Der Innenraum der Apparatur war mit einem milchig trüben Schleier überzogen. Der gebildete Feststoff war schwer löslich (Lösungsmittel: Aceton, Wasser, DMSO, DCM) und konnte aus der Apparatur nur mechanisch entfernt werden. Der Katalysator lag optisch unverändert vor.

Die GC-Analyse des Rohprodukts von Variante A ergab eine Zusammensetzung von 5,9 Gew.-% Propionsäure (entspr. 39.3 g), 2,9 Gew.-% Mercaptoethanol (entspr. 16,2 g), 57,6 Gew.-% Mercaptoethylpropionat (entspr. 293,3 g, 72,6 % der theoretischen Ausbeute) und 4,7 FI-% Hochsieder. Die GC-Analyse des Rohprodukts von Variante B ergab eine Zusammensetzung von 1,2 Gew.-% Propionsäure (entspr. 6.3 g), 0,9 Gew.-% Mercaptoethanol (entspr. 4,6 g), 32,2 Gew.-% Mercaptoethylpropionat (entspr. 170,1 g, 84,5 % der theoretischen Ausbeute) und 0,84 FI-% Hochsieder. Die GC-Analyse des Rohprodukts von Variante C ergab eine Zusammensetzung von 14,8 Gew.-% Propionsäure (entspr. 75,6 g), 7,5 Gew.-% Mercaptoethanol (entspr. 39,7 g), 7,8 Gew.-% Mercaptoethylpropionat (entspr. 41,4 g, 20,6 % der theoretischen Ausbeute). Eine sinnvolle Mengenbestimmung der Hochsieder war aufgrund der massiven Bildung von unlöslichen Feststoffen nicht möglich.

Selbst bei der hohen Verdünnung der Edukte mit 25 Gew.-% Toluol gelang es nicht die Bildung von Hochsieder-Nebenprodukten bei der homogen säurekatalysierten Veresterung zu vermeiden Erst bei sehr hoher Verdünnung der Edukte mit 150 Gew.-% Toluol konnte im Fall der homogen säurekatalysierten Veresterung die Bildung von Hochsieder-Nebenprodukten weitgehend vermieden werden (Variante B). Doch selbst bei einer solch hohen Verdünnung mit Toluol kam im Fall der heterogen katalysierten Veresterung zu einer massiven Bildung von unerwünschten unlöslichen Nebenprodukten (Variante C).

Durch Verdünnung der Edukte mit Schleppmittel lässt sich die Bildung unerwünschter Nebenprodukte bei der säurekatalysierten Veresterung von Carbonsäuren mit Mercaptoalkylalkoholen nur vermeiden, wenn sehr hohe Verdünnungen eingestellt werden und auch nur bei bestimmten Säurekatalysatoren.

### Beispiel 2

Veresterung von Propionsäure mit Mercaptoethanol als Reaktivdestillation mit Verweilzeitbehälter und ohne Zusatz von Säurekatalysator

Der Zulauf zum Verweilzeitbehälter einer kontinuierlichen Laboranlage (schematische Darstellung in Fig. 1) betrug 25 g/h Propionsäure und 28 g/h Mercaptoethanol. Der Verweilzeitbehälter (V) wurde extern beheizt (T_{V} = 115 °C). Der Kopfdruck der Kolonne (K) betrug 284 mbar. Dabei stellte sich ein Temperaturprofil (Sumpf: 138 °C und Kopf: 39 °C) über die Kolonne ein. Der Verweilzeitbehälter hatte einen Füllstand von etwa 1 bis 1,5 I. Vom Verweilzeitbehälter wurde ein Strom des Reaktionsgemisches mit 0,15 bis 0,40 l/h in die Kolonne geführt. Ein an Veresterungsprodukt abgereichertes Reaktionsgemisch, umfassend die unumgesetzte Edukte (Propionsäure und Mercaptoethanol), wurden als Seitenabzug aus der Kolonne abgeführt und dem Verweilzeitbehälter zurückgeführt. Reaktionswasser wurde über Kopf der Kolonne abgetrennt. Das Veresterungsprodukt, Mercaptoethylpropionat, wurde über Sumpf der Kolonne gewonnen. Der Sumpfaustrag enthielt laut GC-Analyse zu über 90 FI-% Mercaptoethylpropionat.

## Patentansprüche

1. Verfahren zur Herstellung von Mercaptoalkylcarboxylaten aus den Edukten Carbonsäure und Mercaptoalkylalkohol durch Veresterung in Abwesenheit von zusätzlichem wirksamen Säurekatalysator durchgeführt wird,
wobei besagter zusätzlicher wirksamer Säurekatalysator eine Menge einer Säure ist, die eine Erhöhung der Geschwindigkeit der besagten Veresterung um mindestens 200 % gemittelt über den gesamten Veresterungsansatz bei ansonsten identischen Veresterungsbedingungen bewirkt, verglichen mit der Veresterung der Carbonsäure mit dem Mercaptoalkylalkohol ohne zusätzliche Säure, und wobei die Veresterung als Reaktivdestillation durchgeführt wird, **dadurch gekennzeichnet, dass**
a) die Edukte Carbonsäure und Mercaptoalkylalkohol als kontinuierlicher Strom der Reaktionszone einer Rektifikationskolonne zugeführt werden,
b) die Edukte in der Rektifikationskolonne zu den Reaktionsprodukten Mercaptoalkylcarboxylat und Reaktionswasser reagieren,
c) das Reaktionsgemisch in der Rektifikationskolonne destillativ aufgetrennt wird, und
d) eine Mercaptoalkylcarboxylat enthaltende Fraktion über Sumpf und eine Reaktionswasser enthaltende Fraktion über Kopf aus der Rektifikationskolonne abgeführt werden.

2. Verfahren zur Herstellung von Mercaptoalkylcarboxylaten aus den Edukten Carbonsäure und Mercaptoalkylalkohol durch Veresterung in Abwesenheit von zusätzlichem wirksamen Säurekatalysator durchgeführt wird,
wobei besagter zusätzlicher wirksamer Säurekatalysator eine Menge einer Säure ist, die eine Erhöhung der Geschwindigkeit der besagten Veresterung um mindestens 200 % gemittelt über den gesamten Veresterungsansatz bei ansonsten identischen Veresterungsbedingungen bewirkt, verglichen mit der Veresterung der Carbonsäure mit dem Mercaptoalkylalkohol ohne zusätzliche Säure, und wobei die Veresterung als Reaktivdestillation in Verbindung mit einem Verweilzeitbehälter durchgeführt wird, **dadurch gekennzeichnet, dass**
a) die Edukte, Carbonsäure und Mercaptoalkylalkohol, als kontinuierliche Ströme dem Gesamtreaktionsraum, umfassend den Reaktionsraum des Verweilzeitbehälters und die Reaktionszone der Rektifikationskolonne, zugeführt werden,
b) die Edukte in dem Gesamtreaktionsraum in Teilumsatz zu Mercaptoalkylcarboxylat und Reaktionswasser reagieren,
c) das Reaktionsgemisch aus Schritt b), umfassend Mercaptoalkylcarboxylat und Reaktionswasser als Produkte der Veresterung sowie unumgesetzte Edukte, vom Verweilzeitbehälter der Rektifikationskolonne zugeführt wird,
d) das Mercaptoalkylcarboxylat und das Reaktionswasser in der Rektifikationskolonne destillativ aus dem Reaktionsgemisch abgetrennt und über Sumpf bzw. über Kopf aus der Rektifikationskolonne abgeführt werden, während produktabgereichertes Reaktionsgemisch als Seitenabzug der Rektifikationskolonne abgeführt wird, und
e) das als Seitenabzug der Rektifikationskolonne abgeführte produktabgereichertes Reaktionsgemisch aus Schritt d) in den Verweilzeitbehälter zurückgeführt wird.

3. Das Verfahren nach Anspruch 1 oder 2, wobei die Veresterung durchgeführt wird in Gegenwart von maximal 5 Gew.-% Schleppmittel bezogen auf die Gesamtmenge der der eingesetzten Edukte Carbonsäure und Mercaptoalkylalkohol.

4. Das Verfahren nach einem der Ansprüche 1 bis 3, **dadurch gekennzeichnet, dass** die Veresterung bei einer Temperatur von 80 bis 200 °C durchgeführt wird.

5. Das Verfahren nach einem der Ansprüche 1 bis 4, **dadurch gekennzeichnet, dass** als Edukt eingesetzten Carbonsäuren 2 bis 8 C-Atome und eine einzige Carbonsäuregruppe haben.

6. Das Verfahren nach einem der Ansprüche 1 bis 5, **dadurch gekennzeichnet, dass** die als Edukt eingesetzten Mercaptoalkylalkohole 2 bis 4 C-Atome, eine einzige Alkoholgruppe und eine einzige Thiolgruppe haben.

## Claims

1. A process for preparing mercaptoalkyl carboxylates by esterification from the starting materials carboxylic acid and mercaptoalkyl alcohol in the absence of an additional active acid catalyst, where said additional active acid catalyst is an amount of an acid which brings about an increase in the rate of said esterification by at least 200% averaged over the total esterification mixture, under otherwise identical esterification conditions, compared to the esterification of the carboxylic acid by means of the mercaptoalkyl alcohol without additional acid, and where the esterification is carried out as reactive distillation, wherein
a) the starting materials carboxylic acid and mercaptoalkyl alcohol are fed as a continuous stream into the reaction zone of a rectification column,
b) the starting materials react in the rectification column to form the reaction products mercaptoalkyl carboxylate and water of reaction,
c) the reaction mixture is fractionally distilled in the rectification column and
d) a mercaptoalkyl carboxylate-comprising fraction is discharged at the top and a fraction comprising water of reaction is discharged at the top of the rectification column.

2. A process for preparing mercaptoalkyl carboxylates by esterification from the starting materials carboxylic acid and mercaptoalkyl alcohol in the absence of an additional active acid catalyst, where said additional active acid catalyst is an amount of an acid which brings about an increase in the rate of said esterification by at least 200% averaged over the total esterification mixture, under otherwise identical esterification conditions, compared to the esterification of the carboxylic acid by means of the mercaptoalkyl alcohol without additional acid, and where the esterification is carried out as reactive distillation in combination with a residence vessel, wherein
a) the starting materials, viz. carboxylic acid and mercaptoalkyl alcohol, are fed as continuous streams to the overall reaction space, comprising the reaction space of the residence vessel and the reaction zone of the rectification column,
b) the starting materials react in the overall reaction space so as to convert them partly into mercaptoalkyl carboxylate and water of reaction,
c) the reaction mixture from step b) comprising mercaptoalkyl carboxylate and water of reaction as products of the esterification and also unreacted starting materials is fed from the residence vessel to the rectification column,
d) the mercaptoalkyl carboxylate and the water of reaction are separated off by distillation from the reaction mixture in the rectification column and are discharged at the bottom or at the top of the rectification column while product-depleted reaction mixture is discharged as side offtake stream from the rectification column and
e) the product-depleted reaction mixture discharged as side offtake stream from the rectification column in step d) is recirculated to the residence vessel.

3. The process according to claim 1 or 2, wherein the esterification is carried out in the presence of not more than 5% by weight of entrainer, based on the total amount of the starting materials carboxylic acid and mercaptoalkyl alcohol used.

4. The process according to any of claims 1 to 3, wherein the esterification is carried out at a temperature of from 80 to 200°C.

5. The process according to any of claims 1 to 4, wherein carboxylic acids used as starting material have from 2 to 8 carbon atoms and a single carboxylic acid group.

6. The process according to any of claims 1 to 5, wherein the mercaptoalkyl alcohols used as starting material have from 2 to 4 carbon atoms, a single alcohol group and a single thiol group.

## Revendications

1. Procédé pour la préparation de carboxylates de mercaptoalkyle à partir des produits de départ acide carboxylique et alcool mercaptoalkylique par estérification en l'absence d'un catalyseur acide actif supplémentaire, ledit catalyseur acide actif supplémentaire étant une quantité d'acide qui provoque une augmentation de la vitesse de ladite estérification d'au moins 200% en moyenne sur l'ensemble de la charge d'estérification à des conditions d'estérification pour le reste identiques, par rapport à l'estérification de l'acide carboxylique avec l'alcool mercaptoalkylique sans acide supplémentaire, et l'estérification étant réalisée sous forme de distillation réactive, **caractérisé en ce que**
a) les produits de départ, acide carboxylique et alcool mercaptoalkylique, sont acheminés sous forme de flux continu vers la zone de réaction d'une colonne de rectification,
b) les produits de départ réagissent dans la colonne de rectification en produits de réaction carboxylate de mercaptoalkyle et eau de réaction,
c) le mélange réactionnel est séparé dans la colonne de rectification par distillation et
d) une fraction contenant du carboxylate de mercaptoalkyle est évacuée via le fond et une fraction contenant de l'eau de réaction est évacuée via la tête de la colonne de rectification.

2. Procédé pour la préparation de carboxylates de mercaptoalkyle à partir des produits de départ acide carboxylique et alcool mercaptoalkylique par estérification en l'absence d'un catalyseur acide actif supplémentaire, ledit catalyseur acide actif supplémentaire étant une quantité d'acide qui provoque une augmentation de la vitesse de ladite estérification d'au moins 200% en moyenne sur l'ensemble de la charge d'estérification à des conditions d'estérification pour le reste identiques, par rapport à l'estérification de l'acide carboxylique avec l'alcool mercaptoalkylique sans acide supplémentaire, et l'estérification étant réalisée sous forme de distillation réactive en association avec un récipient de temps de séjour, **caractérisé en ce que**
a) les produits de départ, acide carboxylique et alcool mercaptoalkylique, sont acheminés sous forme de flux continus vers l'espace total de réaction, comprenant l'espace de réaction du récipient de temps de séjour et la zone de réaction de la colonne de rectification,
b) les produits de départ dans l'espace total de réaction réagissent avec une conversion partielle en carboxylate de mercaptoalkyle et en eau de réaction,
c) le mélange réactionnel de l'étape b), comprenant le carboxylate de mercaptoalkyle et l'eau de réaction comme produits de l'estérification ainsi que des produits de départ non transformés, est acheminé du récipient de temps de séjour vers la colonne de rectification,
d) le carboxylate de mercaptoalkyle et l'eau de réaction sont séparés par distillation du mélange réactionnel dans la colonne de rectification et évacués de la colonne de rectification via le fond ou via la tête, alors que le mélange réactionnel appauvri en produit est évacué sous forme de soutirage latéral de la colonne de rectification, et
e) le mélange réactionnel appauvri en produit, évacué sous forme de soutirage latéral de la colonne de rectification de l'étape d) est recyclé dans le récipient de temps de séjour.

3. Procédé selon la revendication 1 ou 2, l'estérification étant réalisée en présence d'au maximum 5% en poids d'agent d'entraînement par rapport à la quantité totale des produits de départ utilisés, acide carboxylique et alcool mercaptoalkylique.

4. Procédé selon l'une quelconque des revendications 1 à 3, **caractérisé en ce que** l'estérification est réalisée à une température de 80 à 200°C.

5. Procédé selon l'une quelconque des revendications 1 à 4, **caractérisé en ce que** les acides carboxyliques utilisés comme produit de départ présentent 2 à 8 atomes de carbone et un seul groupe acide carboxylique.

6. Procédé selon l'une quelconque des revendications 1 à 5, **caractérisé en ce que** les alcools mercaptoalkyliques utilisés comme produit de départ présentent 2 à 4 atomes de carbone, un seul groupe alcool et un seul groupe thiol.
